# EUROPEAN PATENT SPECIFICATION

(11) **EP 0 342 701 B1**
(45) Date of publication and mention of the grant of the patent: **09.03.1994**
(21) Application number: 89109078.9
(22) Date of filing: 19.05.1989
(51) Int. Cl.: A61B 10/00, A61F 5/44, B01L 3/00, G01N 1/00, A61G 9/00

(54) **Integrity preserving and determining urine sample collection apparatus**
Vorrichtung zum Sammeln von Urinproben mit Reinheitsbewahrungs- und -feststellungsmitteln
Dispositif pour collecter des échantillons d'urine avec des moyens pour en préserver et déterminer la pureté

(30) Priority: 19.05.1988 US 195660
(43) Date of publication of application: 23.11.1989
(73) Proprietor: FRANKLIN DIAGNOSTICS, INC., Morristown, N.J. 07960 (US)
(72) Inventor: Ehrenkranz, Joel R.L., New Jersey 07976 (US)
(74) Representative: LOUIS, PÖHLAU, LOHRENTZ & SEGETH

(56) References cited:
- EP-A- 0 296 799
- DE-A- 2 839 547

## Description

### Field of the Invention

The invention relates to an apparatus for determining if a urine specimen is fresh and unadulterated and for preventing tampering with the urine specimen after it has been collected.

### Description of Related Art

Drug use is generally recognized as a significant contributory factor in the current rise of accidents. Employers, government organizations, and others are increasingly using drug-screening and freedom from drugs as conditions of employment. There are three major problems. First, the collection and testing of urine samples must be simple and cost-effective. Second it has been felt necessary to monitor the taking of urine samples to guarantee the integrity of the test results. Third, the testing aspect, especially coupled with monitoring, has raised concerns about possible invasion of privacy. Accordingly, a need is recognized for a urine sample collector that is simple, straightforward, minimizes the invasion of an individual's privacy, and maximizes the integrity of the sample of urine taken. On Monday, April 11, 1988, the Federal Government issued its final guidelines on Federal Workplace Drug Testing Programs in Volume 53, No. 69 of the Federal Register, pages 11970-11989.

The prior art discloses a few urine measuring devices incorporating a temperature sensor therein. Perhaps most relevant of that group is U.S. Patent 4,564,299 entitled Body Liquid Temperature Measuring Device issued to Joel R. L. Ehrenkranz who is also the inventor of the device described in this disclosure. A chemical melting point thermometer is located in the bottom of the urine collecting receptacle. The primary purpose of the invention is to determine when a human female is close to ovulation.

The patent literature also describes devices, in very different contexts, which employ spill prevention devices in urine collecting embodiments. The purpose of those devices is to prevent spillage after the urine is collected and prior to its being destroyed. For example, U.S. Patents 3,568,218 and 4,457,314 disclose anti-spill devices in two different types of urine collectors. The device in U.S. Patent 4,457,314 is described as an "anti-back flow mechanism" and is employed only to prevent spillage of the contents. Other U.S. Patents that disclose mechanisms for the purpose of preventing spillage include: 3,928,875; 4,059,124; 3,586,041 and 3,734,154.

Another class of urine collectors involve those designed to collect mid-stream urine samples. For example, U.S. Patent 4 494 581 discloses a mid-stream urine collection device which describes, in Fig. 7 thereof, a mechanism in which a floating cork closes a flap after an initial sample of urine has been obtrained so that the remaining specimen to be collected will be from the mid-stream. Other possibly relevant mid-stream urine collection devices include those described in the following U.S Patents: 4 064 760; 4 221 295 and 4 569 090.

Lastly, the following U.S.Patents are cited as being of only general possible relevance: 4 396 113; 4 211 749, 4 443 896 and 4 466 445.

Insofar as understood, none of the prior art addresses the problems inherent in a drug testing program where the drug sample collecting device has to be virtually tamper-proof, easy to use, and maintain the individual's right of privacy.

From the EP-A 0 296 799, which is a document falling under the terms of Article (54)3 EPC, there is known a urine specimen collector which has a bowl member with a threaded bottom opening for attachment of a container. A cover member is pivotally attached to the handle of the bowl and has a cylindrical boss with an opening in line with the bottom opening. The cover serves as a splash gujard. It terminates short of a lower lip of the bowl to allow for overflow.

To detect spurious specimens a thermally sensitive detector is mounted inside the container or on the outer surface thereof to indicate, preferably by irreversible change of color, temperatures below and above blood heat.

### SUMMARY OF THE INVENTION

Briefly described, the invention comprises a urine collection device specifically appropriate for use in drug screening programs. A urine receiving reservoir includes a temperature measuring device, such as a chemical type thermometer, located in a chamber for documenting the freshness of the urine sample immediately after voiding. Urine enters the reservoir through a channel near the center of the device. In the preferred embodiment, the channel includes a specific gravity sensitive mechanism which only passes urine specimen into the collector if the urine specimen is within the correct range of acceptable specific gravity levels. In other words, if a sample of urine is adulterated with water, the reservoir will not accept the urine sample. Also the device is constructed in such a way that as urine fills the reservoir air esapes through vent holes into the colletion bowl. The air holes are of sufficiently small diameter and the center channel is of sufficient depth such that when the urine collector is inverted substantially all of the urine sample remains in the collector. Thus the urine sample can be removed from the collector in only a tamper-evident way.

The inside of the reservoir cap which resides on the base of the collector until the sample is taken includes chemical reagent stations to further discourage or detect tampering or adulteration. One reagent station can determine if the urine sample has been watered down by detecting the specific gravity of the sample. A second reagent station measures the pH of the urine. A third station includes a chemical reagent that helps determine if an enzyme poison, like bleach, dye, detergent or antibody has been added to the urine sample in order to disguise the presence of drugs such as cannabis, cocaine, opiates, amphetamines, sedatives and hallucinogenics.

Because the urine collector is virtually tamper-proof, it eliminates or greatly reduces the need to have the voiding procedure humanly monitored by another individual. This in turn increases the individual's privacy while also guaranteeing that the employer has a fresh, unadulterated, high integrity urine sample.

These and other features of the invention will be more fully understood by reference with the following drawings.

### BRIEF DESCRIPTION OF THE DRAWINGS

Figure 1 is a perspective view of the preferred embodiment of the invention.

Figure 2 shows an exploded view of the receptacle.

Figure 3A is a side elevation of the collector.

Figure 3B is a cross-sectional view taken along lines B-B of Figure 3D.

Figure 3C is another cross-sectional view taken along lines C-C of Figure 3D.

Figure 3D is a top plan view of the collector.

Figure 3E is a bottom plan view of the collector.

Figure 4A illustrates the cap top.

Figure 4B illustrates the cap bottom.

Figure 5 illustrates the collector inverted showing the manner in which the urine sample is retained.

### DETAILED DESCRIPTION OF THE INVENTION

During the course of this description like numbers will be used to identify like elements according to the different figures which illustrate the invention.

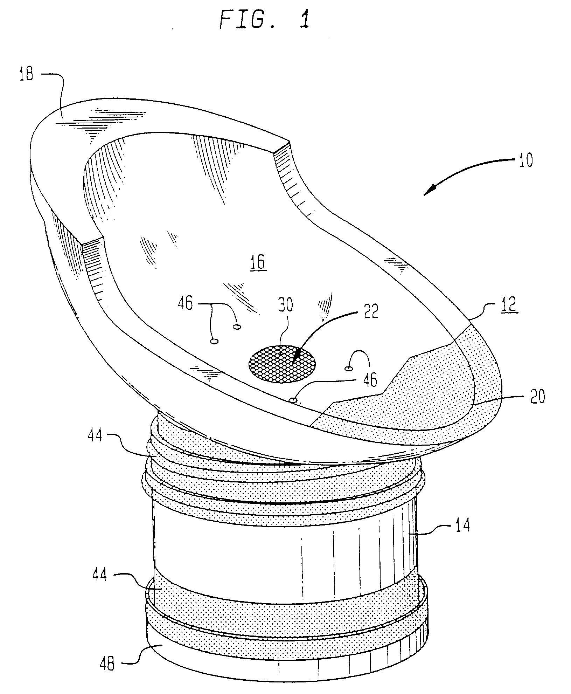

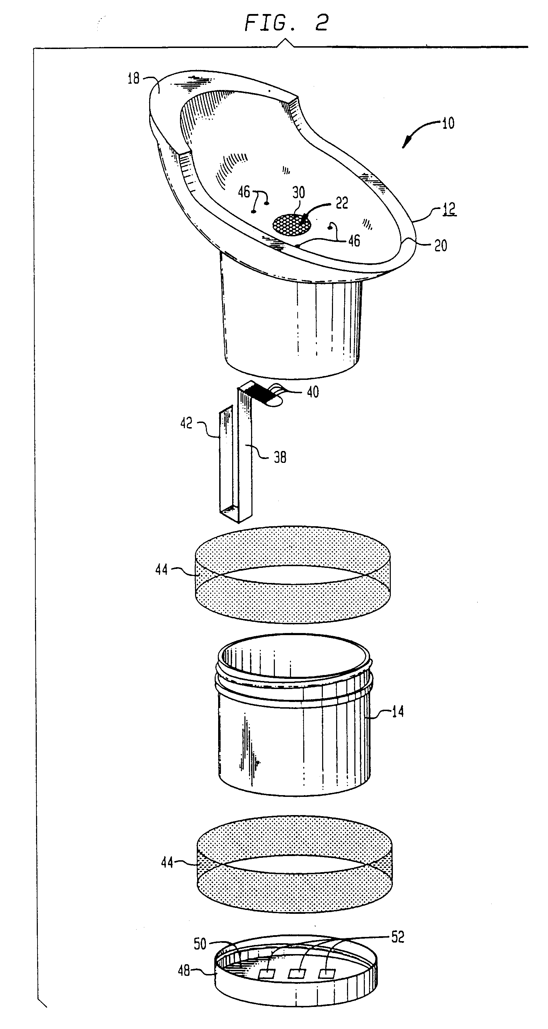

Figures 1 and 2 illustrate the preferred embodiment of the collector 10, which includes a generally elliptical collection apparatus 12 and cylindrical reservoir jar 14. The collection apparatus 12 includes a collection bowl 16 having both a raised portion 18 which acts as both a pouring spout and an anti-splatter shield and a lower rear rim 20. Reservoir jar 14 is intended to hold a urine sample in the range of 60 to 120 cc, with a preferred total capacity of approximately 120 CC.

The accuracy of the invention 10 depends upon minimizing temperature variations due to surface cooling, or heat conducted from the base of the container. Accordingly the base portion of the collection apparatus 12 which is the reservoir jar 14 is of restricted surface area as compared to the upper collection bowl portion 12. Cap 48 at the bottom of the reservoir jar 14 is provided in part to provide additional insulation from ambient temperatures. After use, the cap 48 can be affixed to the top of reservoir jar 14 for storage if the sample is to be retained. Collector 12 also includes a urine input aperture 22 which permits urine to travel from collection bowl 16 into reservoir jar 14.

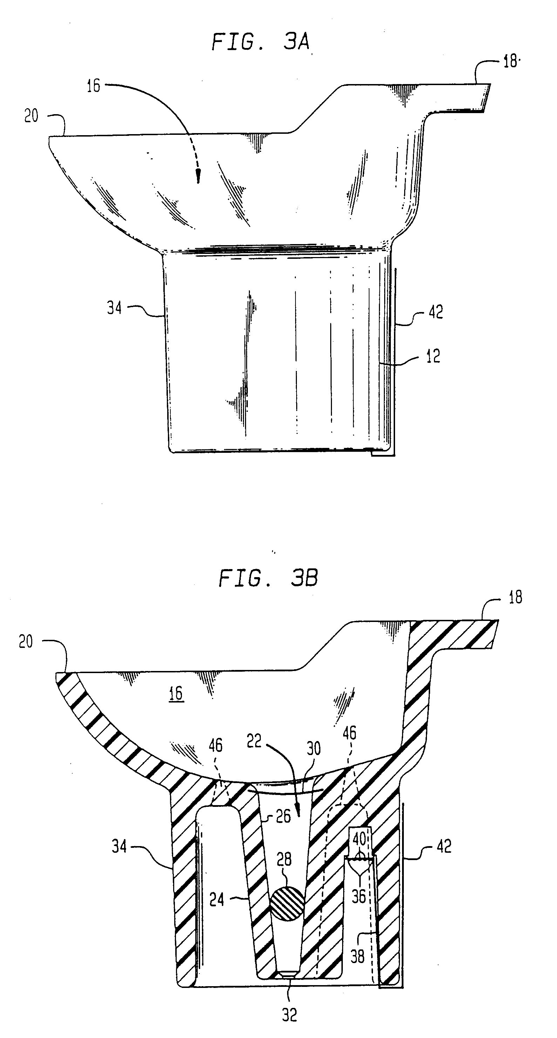

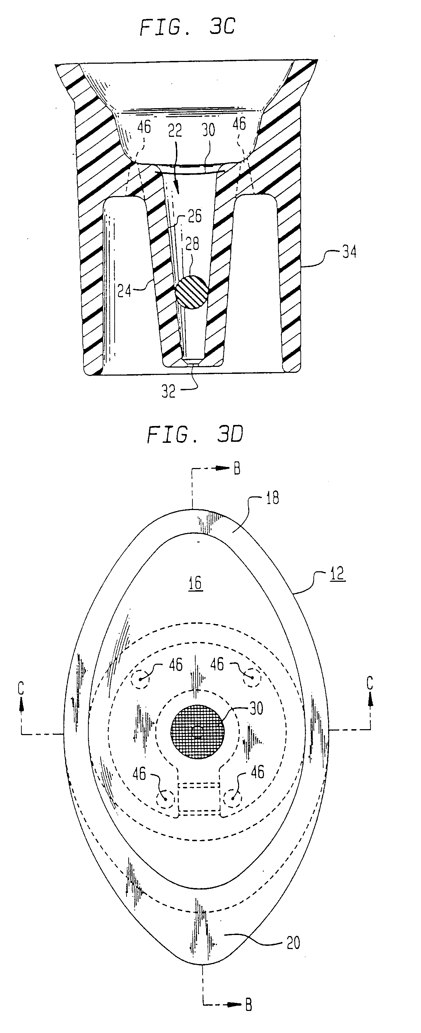

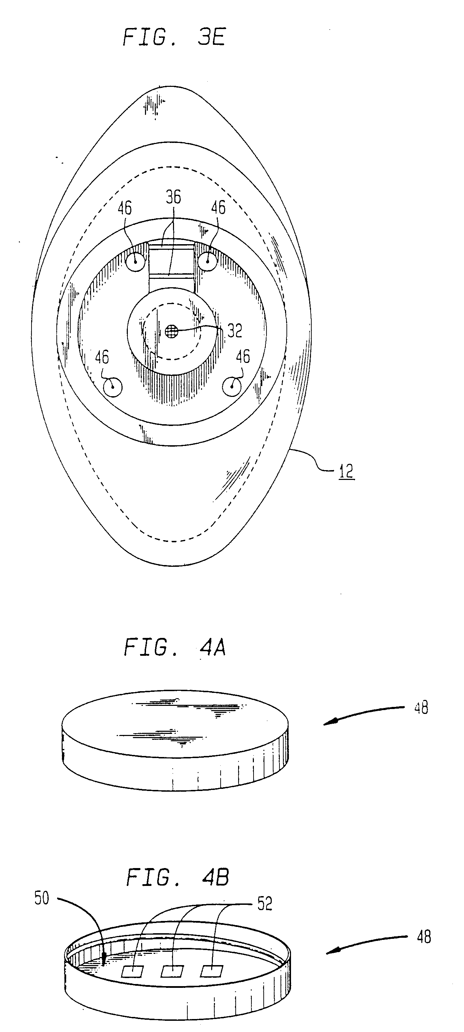

Figures 3A through 3E illustrate the preferred embodiment of collector 12. In operation urine enters collection bowl 16 from the donor and exists bowl 16 through aperture 22 into tapered channel 26 of urine input tube 24. Tapered channel 26 is tapered in such a way as to support ball 28 at approximately the mid-point between urine input aperture 22 and urine exit aperture 32. The tapered channel 26 preferably has an entrance diameter of approximately 12.7 mm (1/2 inch) and an exit diameter of approximately 9.5 mm (3/8 inch). Ball 28 in combination with urine input tube 24, tapered channel 26, and the cage 30 on top of channel 26 which keeps ball 28 in the channel is important to the operation of invention 10. Ball 28 has a specific gravity between 1.001 and 1.002. Thus ball 28 acts like a specific gravity sensitive valve which only permits a urine sample to flow into reservoir jar 18 if the urine sample is within a desired range of specific gravity. Ball 28 is kept in channel 26 by cage 30 at the top and the restrictive taper of channel 26 toward aperture 32 at the bottom. The purpose of this first stage of invention 10 is only to collect specimens of urine that fall above a predetermined range of specific gravity. It is useful to collect urine in this fashion in order to prevent subjects from watering their urine down thereby diluting the accuracy of the test results. Normal urine has a specific gravity of 1.003-1.040. Accordingly, the ball 28 with a specific gravity of 1.001 -1.002 only floats in urine having a specific gravity of 1.002 or greater. Accordingly, adulterated urine samples with a specific gravity less than 1.001 do not get collected in reservoir jar 14.

If urine 60 successfully flows through the ball valve 28, then it collects in reservoir jar 14. Collector 12 has a cylindrical wall base 34 extending below collection bowl 16. Inside the wall 34 at the upper inside exists pedestal 36 for supporting chemical thermometer 38. While a pedestal is illustrated in the preferred embodiment, it will be understood that the thermometer 38 may also be adhesively affixed to the side wall 34 or affixed by either means. This is the second stage of urine testing. It has been found that there is reliable discrimination on the basis of temperature between fresh and previously collected urine samples. Other experiments where the urine sample has been adulterated by the addition of chemicals takes the temperature outside the range or a normal freshly voided urine sample. The temperature range for a normal freshly voided urine sample is 35,8° CC to 38° C (96.4° F to 100.4° F).

Thus chemical reagent thermometer 38 takes and stores the temperature of the urine sample until it can be read. (It stores this information in a chemical dot array 40 at the end of thermometer 38). Thermometer 38 can be removed from the collection apparatus 12 by pulling on handle 42 or, if attached to the inside of the jar, can remain in specimen.

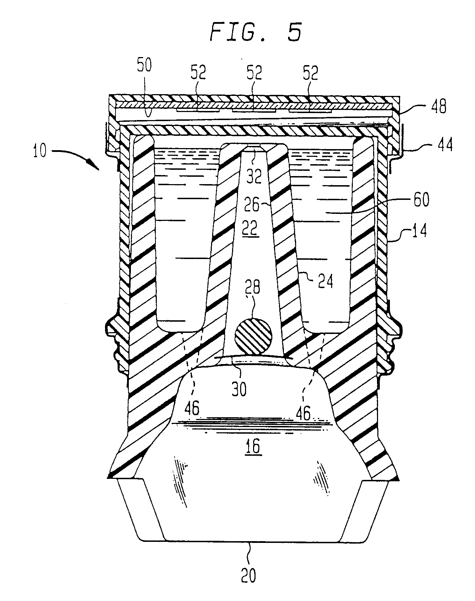

Collector 12 is attached to reservoir jar 14 through the use of tamper-evident tape 44 which attaches to the upper outside surface of reservoir jar 18 and the lower outside surface of collection bowl 16. Tamper-evident tape 44 both seals the collector 12 to the reservoir jar 14 and provides a detectable means of determining if the collector 12 has been partially or completely separated from reservoir jar 18. Because the tape 44 will provide a substantially air-tight seal it is necessary to have a means to permit air to escape, i.e. vent, from reservoir jar 14 as it fills with urine. Air vent holes 46 through collection bowl 16 provide air escape means from the reservoir jar 14. These air holes 46 are of sufficient diameter and number that they will permit air to escape but because of the surface tension of the urine will not permit the urine to escape if the collector 12 is inverted as shown in Figure 5. Air vent holes 46 should be as small as possible, i.e smaller than 0.8 mm (1/32") and preferably 0.4 mm (1/64").

Reservoir jar 14 is a standard urine sample collection jar such as those available from Lerman Container Corporation, 10 Great Hill Road, Naugatuck, CT 06770. Prior to analysis of the sample, cap 48 is removably attached to the bottom of reservoir jar 14. Cap 48 and liner 50 are also available from the Lerman Container Corporation. The cap 48 could be attached by either friction fit to the bottom of reservoir jar or by tamper-evident tape 44.

Figures 4A and 4B show the cap 48. Cap 48 represents the third stage of testing. Cap 48 includes an insulation liner 50 inside the cap for the purpose of insulating the urine sample from the ambient temperature when the cap 48 is on the base of the reservoir jar 14. Cap 48 also contains reagents 52 which do additional testing of the urine sample. These reagents can test for specific gravity, pH, poison, and other features or adulterants of the urine. The pH normal range of unadulterated urine is 4-8. These solid phase reagents 52 react with the urine samples after the collector 12 has been separated from reservoir jar 14 and cap 48 has been removably attached to the top of the reservoir jar 14. It would, of course, be necessary to destroy the integrity of the tamper-evident tape to accomplish this third stage of testing. The tamper evident tape 44 is preferably applied by shrink wrapping, but may be applied in other ways. The fourth stage of testing, incidentally, would be testing the urine sample for specific drug content.

In summary, the present invention is capable of preventing urine donors from tampering with a sample of urine either by means by substitution, adulteration, or dilution. The sample 60 is protected from substitution because the reservoir cannot be emptied if inverted, as shown in Figure 5, and because the temperature thermometer insures that only freshly voided samples of urine are received in the reservoir. Protection against sample adulteration is insured in part by the pH strip on the bottom of the cap and because many urine adulterants produce exothermic reactions which would cause the thermometer inside the reservoir to indicate that the sample had exceeded an acceptable temperature threshold. Lastly, protection against sample dilution is provided, in large part, by the specific gravity sensitive ball valve which only permits non-diluted urine samples having a specific gravity exceeding 1.002 to enter into the reservoir. Additional protection is provided by the use of tamper evident tapes to insure that the reservoir and the collection apparatus are not separated by anyone other than authorized personnel.

## Claims

1. A drug testing urine sample collection apparatus (10) comprising:
a reservoir (14) having a cavity therein for holding a sample of urine; and,
a collection means (12) for collecting urine and transmitting it to said reservoir (14), said collection means (12) including a bowl (16) attachable to said reservoir (14), a tube (24) connected to the bottom of said bowl (16) and including a channel (26) therein for communicating from said bowl (16) into the cavity of said reservoir (14), and air escape means (46) in said bowl (16) which comprises air holes of a diameter smaller than 0.8 mm for permitting air to escape from said reservoir (14) as it fills up with urine, however for not permitting the urine to escape if the collector (12) is inverted,
wherein said tube (24) extends sufficiently deep into said reservoir (14) so as to prevent urine from being emptied from said reservoir (14) when said apparatus (10) is inverted.

2. The apparatus of Claim 1 further including:
a temperature detecting means (38) for measuring the temperature of the urine as it is being collected and for providing an indication of the collection temperature for a prolonged period of time after collection thereby serving as an indication that the sample was fresh and unadulterated when collected.

3. The apparatus of Claim 2 wherein said channel (26) is tapered from a large entrance aperture (22) near said bowl (16) to a smaller exit aperture (32) near the discharge end of the tube (24), said apparatus further comprising:
a sphere (28) having a specific gravity in the range of 1.001 to 1.002 and located in said tapered channel (26);
and ,
a keeper means (30) for preventing said sphere (28) from escaping from the large entrance aperture (22) of said channel (26),
wherein said sphere (28) prevents liquid with a specific gravity less than 1.001 from passing into said reservoir (14).

4. The apparatus of Claim 3 further comprising:
tamper-evident tape (44) attachable to said reservoir (14) and said collector means (12).

5. The apparatus of Claim 4 further comprising:
a cap (48) locatable on the bottom of said reservoir (14) including threads thereon for threadably engaging the top of said reservoir (14) when said reservoir (14) is removed from said collection bowl (16).

6. The apparatus of Claim 5 further comprising:
insulation means (50) located on the inside of said cap (48) for preventing heat transfer to and from the base of said reservoir (14).

7. The apparatus of Claim 6 further comprising:
sample adulteration detection means (52) located on the inside of said cap (48) for detecting if said sample has been adulterated.

8. The apparatus of Claim 7 wherein said channel (26) is tapered from a larger entrance aperture (22) approximately 12.7 mm (1/2") in diameter to a smaller exit aperture (32) approximately 9.5 mm (3/8") in diameter.

9. The apparatus of Claim 8 wherein said air escape means comprise air holes (46) approximately 0.4 mm (1/64") in diameter.

10. The apparatus of Claim 9 further comprising:
a sidewall skirt (34) attached to said bowl (16) and extending downwardly into said reservoir (14) beyond said temerature detecting means (38).

## Patentansprüche

1. Vorrichtung zum Überprüfen von Urinproben auf Drogen, mit einer Speichereinrichtung (14), die einen Hohlraum zum Aufbewahren einer Urinprobe aufweist, und mit einer Sammeleinrichtung (12) zum Sammeln von Urin und zum Weiterleiten desselben zur Speichereinrichtung (14), wobei die Sammeleinrichtung (12) eine an der Speichereinrichtung (14) anbringbare Schüssel (16) enthält, ein Rohr (24) an den Boden der Schüssel (16) angeschlossen ist und einen Kanal (26) enthält, der von der Schüssel (16) in den Hohlraum der Speichereinrichtung (14) hinein eine Verbindung bildet, und in der Schüssel (16) eine Entlüftungseinrichtung (46) vorgesehen ist, die Belüftungslöcher mit einem Durchmesser von kleiner als 0.8 mm aufweist, wodurch ein Entweichen von Luft von der Speichereinrichtung (14) ermöglicht wird, wenn sie mit Urin gefüllt wird, wodurch jedoch ein Auslaufen von Urin nicht möglich ist, wenn die Speichereinrichtung (12) umgedreht wird, wobei sich das Rohr (24) ausreichend tief in die Speichereinrichtung (14) hineinerstreckt, so daß ein Entleeren des Urins aus der Speichereinrichtung (14) verhindert wird, wenn die Vorrichtung umgedreht wird.

2. Vorrichtung nach Anspruch 1, gekennzeichnet durch eine Temperaturerfassungseinrichtung (38) zur Messung der Temperatur des Urins während seiner Sammlung und zur Anzeige der Temperatur des gesammelten Urins während einer verlängerten Zeitdauer nach der Urinsammlung, so daß sich eine Anzeige hierüber ergibt, daß die Urinprobe bei ihrer Sammlung frisch und unverfälscht war.

3. Vorrichtung nach Anspruch 2,
**dadurch gekennzeichnet,** daß der Kanal (26) von einer großen Einlassöffnung (22) nahe der Schüssel (16) zu einer kleineren Auslassöffnung (32) nahe dem Auslassende des Rohres (24) verjüngt ist, daß die Vorrichtung eine Kugel (28) aufweist, die ein spezifisches Gewicht im Bereich von 1,001 bis 1,002 besitzt und die im verjüngten Kanal (26) angeordnet ist, und daß eine Rückhalteeinrichtung (30) vorgesehen ist, durch welche ein Herausfallen der Kugel (28) aus der großen Einlassöffnung (22) des Kanals (26) verhindert wird, wobei die Kugel (28) eine Flüssigkeit mit einem spezifischen Gewicht von kleiner als 1,001 daran hindert, in die Speichereinrichtung (14) hineinzurinnen.

4. Vorrichtung nach Anspruch 3, gekennzeichnet durch ein eine Fälschung anzeigendes Band (44), das an der Speichereinrichtung (14) und an der Sammeleinrichtung (12) anbringbar ist.

5. Vorrichtung nach Anspruch 4, gekennzeichnet durch eine Kappe (48), die am Boden der Speichereinrichtung (14) anordenbar ist und ein Gewinde aufweist zum Anschrauben an der Oberseite der Speichereinrichtung (14), wenn die Speichereinrichtung (14) von der Sammlerschüssel (16) entfernt ist.

6. Vorrichtung nach Anspruch 5, gekennzeichnet durch eine Isolationseinrichtung (50) die an der Innenseite der Kappe (48) angeordnet ist, um eine Wärmeübertragung zur und von der Basis der Speichereinrichtung (14) zu verhindern.

7. Vorrichtung nach Anspruch 6, gekennzeichnet durch eine zum Erfassen einer Fälschung vorgesehenen Einrichtung (52), die an der Innenseite der Kappe (48) zum Erfassen einer Fälschung einer Probe angeordnet ist.

8. Vorrichtung nach Anspruch 7,
**dadurch gekennzeichnet,** daß der Kanal (26) von einer größeren Einlassöffnung (22) mit einem Durchmesser von ungefähr 12,7 mm (1/2") zu einer kleineren Auslassöffnung (32) mit einem Durchmesser von ungefähr 9,5 mm (3/8") verjüngt ist.

9. Vorrichtung nach Anspruch 8,
**dadurch gekennzeichnet,** daß die Entlüftungseinrichtung Belüftungslöcher (46) mit einem Durchmesser von ungefähr 0,4 mm (1/64") aufweist.

10. Vorrichtung nach Anspruch 9, gekennzeichnet durch einen Seitenwandsaum (34), der an die Schüssel (16) angebracht ist und sich nach unten in die Speichereinrichtung (14) über die Temperaturerfassungseinrichtung (38) erstreckt.

## Revendications

1. Dispositif (10) destiné à recueillir de l'urine en vue d'y détecter un médicament, du type comprenant :
- un réservoir (14) formant un volume destiné à contenir un échantillon d'urine ; et
- des moyens (12) pour recueillir l'urine et l'acheminer à ce réservoir (14), ces moyens se composant d'une cuvette (16) pouvant être fixée sur le réservoir (14), un tube (24) adapté au fond de la cuvette (16) et dans l'intérieur duquel un canal (26) met en communication la cuvette (16) et le réservoir (14), et des moyens (46) pour l'évacuation de l'air de la cuvette (16), ces moyens consistant en des perforations d'un diamètre inférieur à 0,8 mm pour permettre à l'air de s'échapper du réservoir (14) à mesure qu'il se remplit d'urine, tout en ne permettant pas cependant à l'urine de s'échapper si le collecteur (12) est retourné,
dans lequel le tube (24) pénètre dans le réservoir (14) sufisamment profondément pour empêcher le réservoir (14) de se vider de l'urine quand le dispositif (10) est retourné.

2. Dispositif selon la revendication 1 comprenant au surplus des moyens (58) de détection de la température en vue de mesurer la température de l'urine à mesure qu'elle est recueillie et de fournir une indication sur la température de collecte pendant une période de temps prolongée au-delà de la collecte, de manière à indiquer que l'échantillon était frais et non altéré au moment de la collecte.

3. Dispositif selon la revendication 2, dans lequel le canal (26) est évasé depuis une grande ouverture d'entrée (22) au voisinage de la cuvette (16) jusqu'à une petite ouverture de sortie (32) au voisinage de l'extrémité d'évacuation du tube (24), ledit dispositif comprenant au surplus :
- une sphère (28) ayant une densité de l'ordre de 1,001 à 1.002 logée dans le canal évasé (26), et
- des moyens de retenue (30) destinés à empêcher la sphère (28) de s'échapper par la grande ouverture d'entrée (22) du canal (26), dans lequel la sphère (28) empêche tout liquide ayant une densité inférieure à 1,001 de pénétrer dans le réservoir (14).

4. Dispositif selon la revendication 3, comprenant au surplus une bande de détection de fraude (44) pouvant être fixée au réservoir (14) et au collecteur (12).

5. Dispositif selon la revendication 4, comprenant au surplus un couvercle (48) pouvant être placé sur le fond du réservoir (14) et présentant un filetage pour être vissé sur le haut du réservoir (14) quand ce réservoir est séparé de la cuvette de collecte (16).

6. Dispositif selon la revendication 5, comprenant au surplus des moyens d'isolation (50) situés sur la face interne du couvercle (48) en vue d'empêcher tout transfert thermique vers la base du réservoir (14) et en sens inverse.

7. Dispositif selon la revendication 6, comprenant au surplus des moyens (52) pour la détection d'une altération de l'échantillon, situés sur l'intérieur du couvercle (48) dans le but de détecter si l'échantillon a été altéré.

8. Dispositif selon la revendication 7, dans lequel le canal (26) est évasé depuis une grande ouverture d'entrée (22), ayant un diamètre d'approximativement 12,7 mm, vers une petite ouverture de sortie, ayant un diamètre d'approximativement 9,5 mm.

9. Dispositif selon la revendication 8, dans lequel les moyens pour l'évacuation de l'air sont des trous d'un diamètre d'approximativement 0,4 mm.

10. Dispositif selon la revendication 9, comprenant au surplus une jupe latérale (34) fixée à la cuvette (16) et descendant dans le réservoir (14) au-delà des moyens de détection de température (38).
